# EUROPEAN PATENT SPECIFICATION

(11) **EP 1 589 834 B1**
(45) Date of publication and mention of the grant of the patent: **11.04.2007**
(21) Application number: 04708526.1
(22) Date of filing: 05.02.2004
(51) Int. Cl.: A23L 1/30, A23L 1/29, A23K 1/16, A61K 31/685

(54) **ENTERAL COMPOSITION FOR THE PREVENTION AND/OR TREATMENT OF SEPSIS**
ENTERALPRÄPARAT ZUR VORBEUGUNG GEGEN BZW. BEHANDLUNG VON SEPSIS
COMPOSITION ENTERALE POUR LA PREVENTION ET/OU LE TRAITEMENT D'UNE SEPTICEMIE

(30) Priority: 05.02.2003 EP 03075356
(43) Date of publication of application: 02.11.2005
(73) Proprietor: N.V. Nutricia, 2712 HM Zoetermeer (NL)
(72) Inventor: SPEELMANS, Gelske, NL-6208 LW WAGENINGEN (NL); VRIESEMA, Adrianus Johannes Maria, NL-3994 KR HOUTEN (NL); VREUGDENHILL, Anita Corinne Eugenie, NL-6243 CP GEULLE (NL); BUURMAN, Willem Andries, NL-6245 GB EIJSDEN (NL); GREVE, Johannes Wilhelmus Maria, NL-6212 CX MAASTRICHT (NL); HOFMAN, Zandrie, NL-6721 RH BENNEKOM (NL); KIVIT, Inge, NL-5321 VN HEDEL (NL); HAGEMAN,Robert Johan Joseph, NL-6705 CT WAGENINGEN (NL)
(74) Representative: van Westenbrugge, Andries
(86) International application number: PCT/NL2004/000082
(87) International publication number: WO 2004/068969

(56) References cited:
- EP-A- 1 090 636
- EP-A- 1 106 181
- WO-A-01/84961
- WO-A-90/15609
- WO-A-03/009704
- DE-A- 19 644 518
- US-A- 5 344 822
- WANG X D ET AL: "PHOSPHOLIPIDS PREVENT ENTERIC BACTERIAL TRANSLOCATION IN THE EARLY STAGE OF EXPERIMENTAL ACUTE LIVER FAILURE IN THE RAT" SCANDINAVIAN JOURNAL OF GASTROENTEROLOGY,, vol. 29, no. 12, 1994, pages 1117-1121, XP009013469 ISSN: 0036-5521 cited in the application

## Description

### Technical Field of the Invention

The present invention relates to an enteral composition containing proteins and lipids, and the use thereof in the manufacture of a nutritional composition for the prevention and/or treatment of endotoxaemia, sepsis or bactaeremia.

### Background of the invention

Sepsis is a disorder which occurs when a relatively large amount of micro-organisms or fragments thereof enters the body. It is characterised as a systemic disease associated with the presence and persistence of pathogenic microorganisms or their toxins in the blood. If endotoxins, which are bacterial fragments, are present in the blood, this condition is also referred to as endotoxaemia or endotoxic shock. When the microorganisms which have entered the blood are viable, this condition is also referred to as bacteraemia. Endotoxin, or lipopolysaccharide (LPS) is a component of the outer cell membrane of Gram-negative bacteria. Lipoteichoic acid (LTA) and peptidoglycan (PG) are components of the outer membrane of Gram-positive bacteria that can also give rise to sepsis. The intestines, especially the colon, are a reservoir of LPS and Gram-negative bacteria, such as the common inhabitant *Escherichia coli,* but also of LTA, PG and Gram-positive bacteria. LPS as well as LTA and PG can enter the systemic circulation by direct translocation from the gut or via translocation of Gram-negative and/or Gram-positive bacteria across the intestinal wall.

The presence of Gram-negative and/or positive bacteria and LPS and/or LTA in the gut is of no particular problem for a healthy person. However, in persons or animals with impaired barrier function of the gut, for instance caused by ischaemia, surgery, chronic inflammation, radiotherapy, trauma, use of certain drugs, such as NSAID's or chemotherapeutics, critically ill persons such as persons under intensive supervision, or persons infected by invasive pathogens, LPS, LTA, Gram-positive bacteria and/or Gram-negative bacteria can cross the intestinal wall and reach the circulation. Once these bacteria have entered the system, LPS and/or LTA are released, which is mainly due to a high activity of the phagocytic system (or shedding by the bacteria). This infection process can also occur via the lungs (or during infections) or via peripheral body parts (e.g. after traumata caused by accidents, or during decubitus, during the last phases of some pregnancy disorders (HELLP syndrome) or during transfusions).

The release of LPS and/or LTA in the body can lead to an acute phase response and sepsis if LPS and/or LTA are not properly neutralised. Such improper neutralisation may occur in animals/persons with a compromised immune function, as is often the case after malnutrition, fasting, surgery, ischaemic conditions, severe burn injury, chronic infection, cancer therapy, imparted liver or spleen function, with critically ill persons, and also with persons that have to recover directly after severe surgery.

The acute phase response can be determined by measuring levels of C-reactive protein in blood. C-reactive protein is an acute phase protein in man and an important component of the innate immune system. C-reactive protein activates the classical pathway of complement, which is one of its main mechanisms in providing host defense. It has recently been recognised that C-reactive protein interacts with the cells of the immune system by binding to Fc gamma receptors. It may thus bridge the gap between innate and adaptive immunity and provide an early, effective antibacterial response. Furthermore, as it protects against the damaging inflammatory response to lipopolysaccharide and cytokines, it may prevent the lethal side-effects of bacterial products. Risk of sepsis can be determined by measuring in vivo protein synthesis in cells of the immune system such as T lymphocytes as described in Januszkiewicz et al. Clin. Nutr. 2001, 20(2), 181-182.

Sepsis can lead to multiple organ failure or even death. It is therefore of great importance to find a method to treat, and especially prevent sepsis. Several approaches have been proposed in the prior art to alleviate the symptoms or prevent or treat sepsis.

One approach has been given in WO-A-98/32428, which describes the use of choline in an enteral feeding for the reduction and/or prevention of endotoxin induced injury and mortality. The amount administered is 1.5 to 20 g per day. Choline is administered as choline tartrate.

Another approach has made use of phospholipids such as given in US 5,434,183 which describes phospholipids containing ω-3 fatty acids DHA and EPA in combination with vegetable oil and/or marine oil for anti-inflammatory and/or immunosuppressive effects in the treatment of rheumatoid arthritis and sepsis. This enables the obtainment of a very low level of serum cholesterol and serum triglycerides.

WO-A-96/04916 describes protein and peptide free intravenous injection preparations containing at least one phospholipid in combination with cholanoic acid or cholanoic acid salt for the prophylaxis and therapy of endotoxin related conditions. Optionally a neutral lipid can be added.

JP-A-05320043 describes a lipopolysaccharide scavenger consisting of phospholipids, in particular phosphatidyl choline, cholesterol and saturated fatty acids, in particular myristic acid. These components are converted into liposomes which are used in a solution for intravenous administration for prevention or treatment of ischaemia or tissue injuries after ischaemia. A suitable concentration ratio of cholesterol to phosphatidyl choline to myristic acid is 5-10:5-10:1-5.

US 4,474,773 also describes the administration of the combination of phospholipids, triglycerides, and cholesterol for treating, among others, dysfunctions of the immune system, the administration according to US 4,474,773 being advantageously made intravenously.

The drawback of these compositions of the prior art for treating sepsis is that the formulations are complex, e.g. a liposome. A further drawback is that parenteral administration is intended which poses a greater risk to the patient. There is thus a need for a relatively simple, effective, safe preparation for the prevention and treatment of sepsis.

WO-A-03/009704, with a publication date of 6 February 2003, describes the use of phospholipids, triglycerides and cholesterol as an enteral feeding for the prevention and/or treatment of sepsis, wherein cholesterol concentrations are mentioned of at least 0.5 % by weight of the composition, and wherein' ratios of phospholipids to triglycerides of 0.32 to 0.80 are exemplified. Cholesterol is often linked with damages to the blood arteries and its presence in a product is believed to reduce the consumer acceptance thereof

In DE-A-19.644.518 mixtures of phospholipids and triglycerides are described having ratios of 0.25 - 0.67, which mixtures can be applied as an enteral feeding. As a lipid source arachidonic acid and/or docosahexaenic acid are required herein, which polyunsaturated fatty acids are in general unstable.

WO-A-90/15609 describes the use of phospholipids, preferably an essentially pure phosphatidyl choline containing only omega-6 fatty acids, against bacterial infections, but does not make mention of endotoxaemia and sepsis. The anti-microbial effect is directed to the killing of bacteria by macrophages, the killing resulting in a massive release of endotoxins that is undesirable for the prevention of sepsis.

It has now been found that sepsis, bacteraemia and/or endotoxaemia can be effectively prevented and/or treated by means of an enteral composition comprising proteins and lipids, the lipid fraction containing phospholipids and triglycerides in a specific ratio.

The present invention is based on the finding that LPS and/or LTA are detoxified in the circulation by incorporation into lipoproteins such as LDL (Low Density Lipoprotein), VLDL (Very Low Density Lipoprotein), chylomicrons and HDL (High Density Lipoprotein), in particular chylomicrons. It is believed by the inventors that chylomicrons play an important role in absorbing and transporting lipophilic substances in general such as food components and toxins (e.g. LPS and/or LTA), which can enter the body via the intestine but which can also be formed in case of elimination of remnants of dead bacterial cells in parts of the body. Chylomicrons are released in the gut-associated lymphoid tissue (GALT) and take up LPS and/or LTA that are released after lyses of bacteria in the enterocytes and in particular in the lymph nodes. The chylomicrons are transported from the lymph nodes via the ductus thoracicus to the angulus venosus sinister, which transports the chylomicrons, and other lipoproteins, to the heart, which then transports them to the Reticulo Endothelial System (in particular the spleen) and the liver (Kupffer cells). Chylomicrons can thus also be used as a vehicle for delivery in the liver of lipid soluble substances, thereby preventing losses which can occur from malabsorption.

Maintaining the natural level of chylomicrons, not only in blood plasma, but especially in GALT e.g. over a relatively large part of the length of the gut and during a prolonged time period, ensures that most of the LPS and/or LTA which are released in several locations of the body e.g. in the gut or lungs, can be neutralised, substantially decreasing the risk of locally occurring high levels of LPS and/or LTA.

In view of these findings, it is thus essential according to the invention to administer the combination of proteins and lipids, the lipids comprising phospholipids, and triglycerides, as an enteral composition and not as prescribed by the prior art as an intravenous composition. The combination of proteins and said lipids is digested in the intestine, ensuring a relatively constant release of chylomicrons for a prolonged period of time in GALT since the product is relatively slowly digested. Further, the combination of phospholipids and triglycerides has the advantage that the ingredients used need no specific pretreatments such as liposome formation, which results in an effective product with a relatively low cost price. Further, enteral administration of the composition is simple and safe.

Compared to US 4,474,773 which provides the intravenous administration of phospholipids, triglycerides and cholesterol, it has now been found, as explained above, that the enteral administration favours the formation of chylomicrons in GALT, thereby providing effective prevention and/or treatment of sepsis. In this respect, it is believed that the teaching of US 4,474,773 would be detrimental to such prevention and/or treatment. Indeed, US 4,474,773 provides the stimulation of the immune system by increasing the lymphocyte production. However, a problem encountered with this stimulation is that cytokines are produced, which components are involved in the pro-inflammatory response that underlies sepsis (Intensive Care Med. 2000;26 Suppl. 1:S124-8, Immunomodulatory therapy in sepsis, Kox WJ et al.). This is further confirmed by Inflamm. Res. 1998 May; 47(5):201-10, "The inflammatory basis of trauma/shock associated multiple organ failure", Yao YM et al, which describes that activation of the immune system may produce a generalised inflammation finally leading to sustained inflammation and multiple organ damage. Accordingly, though following the teaching of US 4,474,773, a stimulation of the immune system would be provided, this would nevertheless be detrimental to the treatment and/or prevention of sepsis.

Wang X.D. et al describe in Scand. J. Gastroenterol 1994:1117-1121 that the enteric administration of phospholipids significantly reduced the incidence of bacterial translocation after 90 % hepatectomy in rats. They describe that bacterial translocation under certain conditions may cause sepsis or bacteraemia. It is then concluded that the decrease in bacterial translocation is probably the result of phospholipids nourishing the intestinal mucosa and maintaining the intestinal barrier or by preventing of the barrier function as mucosal surfactant. However, this article makes no mention of chylomicrons, let alone of their relationship with the prevention or treatment of sepsis.

Still another approach has been given by WO-A-01/19356 - or its equivalent EP-A-1.090.636 - which prescribes the enteral administration of the combination of medium chain triglycerides and lipid to prevent sepsis, exemplifying ratios of phospholipids to triglycerides of less than 1. Medium chain triglycerides have not been found effective in the neutralisation of LPS nor in the formation of chylomicrons.

In the compositions comprising phospholipids and triglycerides for the prevention of sepsis according to WO-A-03/009704, DE-A-19.644.518 and WO-A-01/19356 the ratio of these components is such that an excess of triglycerides over phospholipids in the composition is obtained. Such products do not suffer from instability problems as are observed for products having such high ratios of phospholipids to triglycerides as in the present invention.

### Summary of the invention

According to one aspect of the invention, there is provided the use of a composition comprising proteins and lipids, the lipids comprising phospholipids and triglycerides in a weight ratio of phospholipids to triglycerides greater than 1.0, and wherein the composition comprises less than 0.5% by weight of cholesterol or precursors thereof, for the manufacture of a nutritional composition for the treatment and/or prevention of endotoxaemia, sepsis, or bacteraemia by enteral administration.

According to another aspect of the invention, there is provided an enteral composition comprising:
a)- at least 1 % by weight of proteins and/or peptides, and
b)- at least 3.5 % by weight of lipids, the lipids comprising phospholipids and triglycerides in a weight ratio of phospholipids to triglycerides greater than 1.0, and wherein the composition comprises less than 0.5% by weight of cholesterol or precursors thereof, wherein said phospholipids are present in an amount of at most 78 wt% of the lipid fraction, and wherein the triglycerides contain at least 50 wt% long chain fatty acids

These compositions with a ratio of phospholipids to triglycerides greater than 1 can be rendered stable using i) certain combinations of phospholipids, ii) the presence of organic acids and/or iii) pH control during preparation. Moreover, it has been found that when cholesterol is present below 0.5 % by weight in the composition according to the invention, the stability of the composition is further enhanced.

### Detailed description of the invention

Lipids are essential ingredients of the present invention. Typically, the amount of lipids present in the invention composition is of at least 3.5 % by weight of the composition, preferably at least 4 % by weight of the composition, more preferably of at least 6 % by weight of the composition. Preferably, the lipids are present up to a level of 50 % by weight of the composition, more preferably up to a level of 20 % by weight of the composition.

An essential lipid for the purpose of the present invention is a phospholipid. Phospholipids for use herein are selected from phosphatidyl choline, phosphatidyl ethanolamine, phosphatidyl inositol, phosphatidyl serine, phosphatidyl glycerol, phosphatidic acid, source thereof and mixtures thereof.

As a source of phospholipids, lysophospholipids, preparations enriched in a particular phospholipid or containing relatively pure (synthetic) phospholipids can be used. Though after consumption of synthetic phospholipids and even lysophospholipids, chylomicrons are formed in vivo, it is preferred to use natural sources, in particular soy lecithin and egg lecithin. Indeed, these are economical and favoured by consumers but have also been found more stable in finished product.

Preferably, the phospholipids are administered as a mixture of phospholipids, in particular comprising phosphatidyl choline and one or more of phosphatidyl ethanolamine, phosphatidyl inositol, phosphatidyl serine, phosphatidyl glycerol, and phosphatidic acid. More preferably, the composition contains at least phosphatidyl choline and phosphatidyl ethanolamine.

Natural sources typically comprises mixtures of phospholipids. Hence, a preferred soy lecithin for use herein as a natural source of phospholipids contains, based on total phospholipid content, 20 to 40 wt.% phosphatidyl choline and 20 to 33 wt.% phosphatidyl ethanolamine. The amount of negatively charged phospholipids, that is the total amount of phosphatidic acid and/or phosphatidyl serine and/or phosphatidyl inositol and/or phosphatidyl glycerol, present in the soy lecithin, is from 10 to 50 % by weight.

A preferred egg phospholipid extract for use herein as a natural source of phospholipids comprises 73 to 82 wt.% phosphatidyl choline and 15 to 22 wt.% phosphatidyl ethanolamine. The amount of negatively charged phospholipids present in the egg lecithin is less than 2 % by weight.

Typically, phospholipids for the purpose of the present invention will be present in an amount of 23 % to 95 %, preferably of 50 % to 95 % and more preferably greater than 50 %, by weight of the lipid fraction of the composition. Still, it has now also been found that the presence of a high level of phospholipids present in the lipid fraction of the invention composition further improved the prevention and/or treatment of sepsis. Accordingly, it is preferred that phospholipids are used or present at a level of 45 % to 78 %, more preferably of 50 % to 69 wt % of the lipid fraction of the composition; the lipid fraction being preferably represented by the combination of phospholipids and triglycerides.

For improved stability of the invention composition, in particular at high amounts of phospholipids, it is preferred that the composition of the phospholipids is such that the amount of phosphatidyl choline, when present, is less than or equal to 72 % of the total amount of phospholipids present in the composition, preferably less than 50 %, more preferably less than 35 % by weight and most preferably less than 25 % by weight of the total amount of phospholipids present in the composition. The weight percentage of phosphatidyl ethanolamine, when present is preferably below 70 %, more preferably below 50 % of the total amount of phospholipids present in the composition. The weight percentage of negatively charged phospholipids, when present, that is the total amount of phosphatidic acid and/or phosphatidyl serine and/or phosphatidyl inositol and/or phosphatidyl glycerol, is preferably above 5 %, more preferably above 7 %, most preferably above 10 %.

Accordingly, for the purpose of the invention, it is preferred not to use pure egg phospholipid or egg lecithin as sole source of phospholipids in view of their phospholipids content, especially high content of phosphatidyl choline, and low content of negatively charged phospholipids but instead to use mixtures of pure egg phospholipid or egg lecithin with any of the following components or mixtures thereof: soy lecithin or soy phospholipids or lecithins or phospholipids of other origin so that a lower amount of phosphatidyl ethanolamine and phosphatidyl choline and a higher amount of negatively charged phospholipids than found in egg lecithin is obtained.

The fatty acids in the phospholipids in particular comprise less than 30 % docosahexaenoic and/or eicosapentaenoic acid. Phospholipids of the invention are in particular selected from glycerol esterified with long chain fatty acids. With long chain fatty acids, fatty acids are meant with at least 18 carbon atoms. The amount of long chain fatty acids related to the total amount of fatty acids in the phospholipid, should be at least 50 %, preferably more than 60 % and most preferably more than 70 % on molar basis. Preferred long chain fatty acids are stearidonic, palmitic, oleic, α-linolenic, linoleic, gamma-linolenic, conjugated linoleic acid, docosahexaenoic, eicosapentaenoic and arachidonic acid. The amount of myristic acid in the phospholipid fraction is preferably smaller than 40 %, more preferably smaller than 25 wt.%. Medium chain fatty acids which typically include chain length in the range of 6 to 12 carbon atoms are not desired for use herein as the main component of the phospholipid due to their low performance in LPS neutralisation. Accordingly, it is preferred that the phospholipid fraction contains at least 50 % by weight, more preferably more than 70 % by weight and most preferably more than 75 % long chain fatty acyls.

Phospholipids are administered in an amount of 0.008 - 2.0 gram, preferably 0.01 - 2.0 gram, more preferably more than 0.035 gram per kilogram body weight per unit dose. For humans, assuming a body weight of 70 kg, this results in about 0.6 to 140 g per unit dose, preferably about 0.7- 140 g per unit dose, more preferably more than 2.5 g per unit dose. The phospholipids are administered in doses at least once every 24 hours, but preferably every 2-12, more preferably every 3-8 hours or even on a continuous basis. Stated otherwise, the number of doses per 24 hours when not administered on a continuous basis is within the range of 2 to 12, preferably of 2 to 7, with that respect that the daily dosage is 0.008 - 2.0 gram, preferably 0.01 - 2.0 gram, more preferably more than 0.035 gram per kilogram body weight. When on continuous basis, the amount continuously administered over 24 hours constitutes one dose.

The present invention is particularly suited when short term tube feeding is used. Accordingly, for a complete enteral nutrition composition and considering a consumption of 2000 kcal, a daily dosage of phospholipids for short term tube feeding is 12-140 g, preferably 12-100 g, more preferably 12-75 g, even more preferably 16-75 g and most preferably 30-75 g. 16-60 g phospholipids per day is especially preferred for a short term tube feeding. A typical tube feeding therefore contains 12-140 g phospholipids, preferably 12-100 g, more preferably 12-75 g, even more preferably 16-75 g and most preferably 30-75 g per 2000 kcal. 16-60 g per 2000 kcal is especially preferred.

When the composition is used as a supplement, and considering a consumption of 200-600 ml and a lipid content of about 10 %, a daily dosage of phospholipids for a supplement is preferably 2.3-56 g, more preferably 5-50 g. When the composition is used as a supplement and a consumption of 200-600 ml and a lipid content of about 5 % is considered, a daily dosage of phospholipids for a supplement is preferably from 2.3-28 g, more preferably 5-25 g.

Another essential lipid for the purpose of the present invention is a triglyceride. Triglycerides of the invention are in particular selected from glycerol esterified with long chain fatty acids. With long chain fatty acids, fatty acids are meant with at least 18 carbon atoms. The amount of long chain fatty acids related to the total amount of fatty acids in the triglyceride, should be at least 50 %, preferably more than 60 % and most preferably more than 70 % on molar basis in order to observe significant chylomicron formation in vivo. Preferred long chain fatty acids are stearidonic, oleic, α-linolenic, linoleic, gamma-linolenic, conjugated linoleic acid, docosahexaenoic, eicosapentaenoic and arachidonic acid. More preferably, polyunsaturated fatty acids are used which result in more efficient chylomicrons, i.e. in chylomicrons which are more suitable to scavenge LPS and/or LTA. The amount of myristic acid in the triglyceride fraction is preferably smaller than 40 %, more preferably smaller than 25 wt.%. Medium chain triglycerides which typically include chain length of fatty acid in the range of 6 to 12 carbon atoms are not desired for use herein as the main component of the triglycerides due their low performance in the LPS neutralisation as well as their low performance in the chylomicrons formation. Accordingly, it is preferred that the triglycerides fraction contains at least 50 % by weight, more preferably more than 70 % by weight and most preferably more than 75 % of long chain fatty triglycerides. The fatty acids in the triglycerides in particular comprise less than 30 % docosahexaenoic and/or eicosapentaenoic acid.

Preferably, the amount α-linolenic acid in the phospholipid and triglyceride fraction does not exceed 4.5 % of energy of the total food composition, whereas the amount of linoleic acid does not exceed 10 % of energy of the total composition.

Examples of triglycerides are vegetable oils such as soy oil, canola oil, corn oil, olive oil, sunflower oil, sesame oil, safflower oil, wheat germ oil, arachidic oil, evening primrose oil, oils from animal origin such as egg oil, or mixtures thereof, but also marine oils, such as fish oil and algal oil are suitable, optionally mixed with one or more vegetable oils.

Typically, triglycerides for the purpose of the present invention will be present in the lipid fraction of the invention composition in an amount of at least 7 % to less than 77 wt.%. Still, it is preferred when phospholipid is present in a high amount of the lipid fraction that a level of 7 % to 50 wt.% of triglycerides is used or present in the lipid fraction of the invention composition.

The daily dose of triglycerides is from 0.01 to 1.5 g/kg body weight. This results in administration of about 0.7 to 105 g, preferably of about 0.7 to 90 g triglycerides for a diseased person of 70 kg body weight. This is given in separate doses (1 to 12, preferably 2 to 7, per 24 h) or on a continuous base (considered as 1 dose). The dose regimen and amount of triglycerides is chosen in such a way that the amount per unit dose is 0.01 to 1.5 g/kg body weight.

The daily dose of phospholipids and triglycerides is chosen in such a way that it results in either restoring the lipoproteins from low levels to levels observed in healthy well fed persons or even enhancing the levels in a short period of time to 120 % or even above 150 %, compared to a healthy person and/or to enhance the phospholipid/lipoprotein ratio in the blood. Preferably the daily dosage of triglycerides is from 0.01 to 1.5 g/kg body weight. Indeed, it is believed that it is preferred to have a weight ratio which is high in favour of the phospholipids. Chylomicrons (and other lipoproteins) containing a high amount of phospholipids will have a higher capacity in neutralising endotoxin. Furthermore, a high amount of phospholipids will aid in absorption of triglycerides.

The components of the composition of the invention are present in their natural form or as separate ingredients. This means that liposomes are not envisaged by the invention.

In the composition of the invention the weight ratio of phospholipids to triglycerides is greater than 1, more preferably greater than 1.5, and even more preferably greater than 2.

As a source for the combination of phospholipids and triglycerides of the invention, eggs such as egg in the form of egg oil or egg powder can also be used. Eggs, in particular the lipid fraction thereof, already contain the desired components. Depending on the end use of the composition and the type of lipid extract that is used, all or some of the components (phospholipids and triglycerides) need to be supplemented to fall within the ranges indicated, such that the diet of the patients contains the right amounts of these components. It is particularly advantageous if eggs are used of animals having a specific lipid mixture in their feed, which eggs have an increased content of α-linolenic acid, conjugated linoleic acid and docosahexaenoic acid. As mentioned before, pure egg phospholipid or egg lecithin are mixed with any of the following components or mixtures thereof: soy lecithin or soy phospholipids or lecithins or phospholipids of other origin so that a lower amount of phosphatidyl ethanolamine and phosphatidyl choline and a higher amount of negatively charged phospholipids than found in egg lecithin is obtained.

Still another essential component of the invention is a protein. Addition of a protein to the lipid combination will enable the formation of a complete nutrition composition. The administration of a complete nutrition composition is an important requirement for patients with diseases or patients in Intensive Care, as it will enable the delivery of all the necessary macronutrients as well as micronutrients in one composition. Preferably, the protein is selected from milk protein, whey protein, casein protein, soy protein, egg protein, and mixtures of these proteins. The protein may be in the form of intact protein or may be (partly) hydrolysed. Other proteins such as wheat rice, pea and oat proteins, or their hydrolysates, or mixtures thereof, may also be used. Further, if desired, the protein source may include free amino acids.

Protein can advantageously be provided by eggs. Indeed, besides that it contains the suitable components of the invention, eggs also have the advantage that they contain beneficial proteins. The protein fraction of eggs contains immunoglobulins which can interact with bacteria. The presence of immunoglobulins, in particular IgY, together with the components of the invention in the intestine, provides a very efficient system which prevents that bacteria translocate through the intestinal wall.

It is preferred that these immunoglobulins, in particular IgY, have been made suitable to bind to specifically those bacterial strains which form the greatest risk for causing sepsis. Such immunoglobulins can be induced in birds and transferred to eggs according to methods known in the art. A particularly suitable product to be used according to the invention is thus hyperimmunised egg, which can be used as such after a slight pasteurization, which keeps the immunoglobulins predominantly in undenatured and active state.

Preferably, at least 30 % by weight of the proteinaceous material is constituted of proteins and/or peptides having a molecular weight of at least 0.3 kDa, more preferably of at least 0.5 kDa, most preferably of at least 1 kDa, and even most preferably of at least 2 kDa. These proteins have been found particularly beneficial for use in the present invention. Further, use of proteins of higher molecular weight, for example of molecular weight higher than 10 kDa results in a more stable product.

The composition preferably contains at least 0.02 g, preferably 0.07 to 2.5 g, more preferably 0.08 to 2.0 g proteins per per kg body weight per day. For humans, assuming a body weight of 70 kg, this results in at least 1.4 g, preferably 4.9 to 175 g, more preferably 5.6 to 140 g proteins per day. Per unit dose the amount of proteins is at least 0.02 g, preferably 0.07 to 2.5 g, more preferably 0.08 to 2.0 g per kg body weight.

When proteins are used that originate from eggs of hyperimmunised birds, the daily dose of IgY that can be administered is then preferably from 0.2 to 120 mg. Still, as the effectivity of the IgY when combined with invention composition has been found to improve the prevention of the bacteria translocation, the daily dose of IgY that can be administered is more preferably within the range of 0.2 to 800 mg, most preferably of 10 to 600 mg. The dose of this ingredient is preferably 0.1 mg to 10 mg per kg body weight per dose. For humans, assuming a body weight of 70 kg, this results in 7 - 700 mg per unit dose.

The amount for a dose can be determined based on the weight of the subject to be treated. The daily doses can be calculated by multiplying the dose with the number of times that the dose is consumed per day. The dose regime should be adjusted to maintain a high level of chylomicrons in the lymph e.g. by repeating dosing every 2-12 hours, preferably 3-8 hours or even on a continuous basis. For a group of patients tube feeding in the gut is recommended.

A preferred characteristic of the present invention is the composition being substantially free of cholesterol or its precursor thereof. Indeed, it has been found that the presence of cholesterol could be substantially reduced or eliminated without affecting the LPS neutralisation and/or the chylomicrons formation. A further advantage of this substantial reduction level of cholesterol or even elimination is the consumer perception of the product. The product is seen as healthier for the consumer as cholesterol is often linked with damages to the blood arteries, thus further deteriorating the condition of a patient.

By precursor of cholesterol, it is meant cholesteryl esters like those that occur in natural extracts (egg) and synthetic sources such as esters with organic acids, e.g. cholesteryl acetate, hemisuccinate, n-butyrate, oleate, or esters with phospholipids.

Preferably, by "substantially free of cholesterol or precursors thereof", it is meant that the composition comprises less than 0.5 % by weight of cholesterol or precursors thereof, preferably less than 0.2 % by weight and more preferably is free of cholesterol and precursors thereof.

It has also been found that when cholesterol is present in low amounts only or even absent, the best response in terms of stability of the composition, especially when soy lecithin is used, and production of chylomicrons is obtained. A composition comprising egg lecithin sometimes has a tendency to an undesired creaming of the composition. Use of such specific low cholesterol level combined with soy derived phospholipids or even absence of cholesterol or precursors thereof in a composition comprising phospholipids and triglycerides as defined herein has been found to provide a remedy to this problem whilst still providing effective production of chylomicrons.

When present, the dose of cholesterol or equivalents thereof calculated as cholesterol is preferably of less than 2 mg per kg body weight per dose resulting in a dose of less than 0.14 g, preferably less than 0.1 g in humans per unit dose.

The enteral composition can further contain organic acids such as citric acid, malic acid, lactic acid, their salts, or mixtures thereof to further increase the stability of the composition. Preferred organic acids for use herein are polydentate acids such as citric acid, its salt or mixtures thereof. When present, these organic acids are present in the composition in a concentration of at least 0.5 %, more preferably more than 0.6 % most preferably more than 0.8 % up to 2%, more preferably up to 1.5 % by weight of the composition. Based on dry weight of the composition, the amount of organic acids is in the range of more than 2.5 wt.%, preferably more than 3 wt%, and most preferably more than 4 wt.% with a maximum of 10 wt.%.

The enteral composition can also further contain lactic acid bacteria, products of lactic acid bacteria, prebiotics, extra L-glutamine and/or L-arginine, antioxidants, fibres, carbohydrates, polysaccharides, vitamins, minerals such as zinc which will promote the adsorption of lipid-soluble substances, and also other components which are normally present in an enteral feeding.

When present, the carbohydrate may be any suitable carbohydrate or carbohydrate mixture. For example, the carbohydrate may be glucose, sucrose, maltodextrin, modified starch, amylose starch, tapioca starch, corn starch, corn syrup, or fructose, or mixtures thereof. Maltodextrin is preferred if low osmolarity is required.

The composition can contain fat-soluble substances. These are in particular selected from vitamin K (menaquinones), ubiquinones, carotenoids such as vitamin A, specific fatty acids such as conjugated linoleic acid, lipoic acid and vitamin D. Inclusion of one or more of these components in the lipid mixture of the invention effectively decreases the prevalence of sepsis, decreases acute phase response and improves recovery after surgery or trauma.

When they are included in an enteral product together with the lipid mixture of the invention and optionally with zinc salt, lower amounts of these fat soluble substances than those in the prior art can be effective.

It is also preferred to incorporate berberin or extracts of Berberis aristata or Coccinia fenestratum in the preparation in an amount of 10-100, preferably 20-50 mg/kg body weight per dose.

The enteral composition of the invention is preferably used in the prevention and/or treatment of sepsis, bacteraemia and/or endotoxaemia (endotoxic shock) and to delay acute phase response. In particular the composition is used for treatment of patients undergoing major surgery, critically ill patients, patients with Inflammatory Bowel disease (IBD), patients with HELLP syndromes, patients with an enhanced risk for bacterial translocation and sepsis in general, in particular those suffering from major trauma, burns, pneumonia, especially caused or complicated by bacteria, decubitus, during radio/chemotherapy or patients having a compromised immune system such as premature infants or people suffering from elderly diseases, or people with an obstructed bile duct.

In case of surgery, it is believed that the risk of endotoxaemia is related to the fact that patients need to fast preoperatively and that after surgery take only little food. As a result, the normal amount of chylomicrons is diminished, which makes them more vulnerable for damage caused by LPS. Generally these patients can take the composition of the invention until about 3 hours before surgery and shortly after surgery, but also the option of taking the composition during a shorter period before surgery is envisaged by the invention. In this case the total lipid level in the composition will typically be in the range of 5 to 70, preferably 10 to 50 g per dose. This dose can be supplied by means of a supplement for oral use, preferably a liquid such as a dairy based drink in a relatively low volume, for example 100 to 700, preferably 150 to 600 ml.

The composition can also be taken by persons that allow intake of a restricted volume, such as persons suffering from anorexia nervosa or persons in the end stages of diseases such as cancer or AIDS, but also many elderly people. In this case the triglyceride level in the composition will be in the range of 0.7 to 30, preferably 2 to 15 g/dose, administered in a relatively low volume.

The composition of the invention can also be used fur the treatment of mammals in general which are susceptible to infections, such as due to irregular feed regimes, e.g. cows and pigs, in particular weaning pigs, where infections can result in lower meat quality. Stress during transport can also result in bacterial translocation in animals.

The enteral composition of the present invention can be an oral or tube feeding, preferably a tube feeding. The tube can be nasooesophagial, nasogastric or nasojejunal, but also gastrostomy or jejustomy tubes are envisaged. The oral feeding can be a complete feeding or a food supplement, in liquid form or as a capsule or powder. Preferred liquid forms are dispersions. Intravenous compositions for the reasons explained hereinbefore are excluded from the invention.

Thus the invention also provides an enteral food containing per dose
8-180 g, preferably 8-140 g, more preferably 8-120 g, even more preferably 12-120 g and most preferably 12-80 g of lipid containing the combination of phospholipids and triglycerides
3-120 g, preferably 3-100 g, more preferably 12-100 g and most preferably 30-90 g of a protein fraction and optionally
1.2-400 g, preferably 5-200 g of a carbohydrate fraction.

A preferred process for making such enteral composition involves separately mixing the lipid ingredients and the other composition ingredients. The pH is preferably adjusted to about 7.0 - 7.2. The lipid ingredients are then incorporated to the mixture preferably under high shear. The blend is then pasteurised and homogenised (preferably carried out at 550 bar and 50 bar). Once homogenised, the pH of the blend is adjusted to a range of 6 to 8 when measured at 20 °C. Indeed, regulation of the pH during pasteurisation to a pH range within the range of 6 to 8, preferably to pH 6.2 was found preferred for obtainment of a composition with optimum stability. A preferred agent for regulation of the pH is a mixture of citrate and citric acid (weight ratio 1:1). However, other regulating agents which have the capacity of binding divalent cations may also be used herein. Once the pH is adjusted, the blend is sterilised.

Accordingly, in a preferred embodiment of the present invention, there is provided a process for making a composition as defined hereinbefore, wherein the process comprises the steps of:
a)-mixing the non-lipid ingredients together and adjusting the pH to a neutral value, pH within the range of 6 to 8;
b)-incorporating the lipid ingredient in the mixture obtained in step a) by mixing;
c)-pasteurising the mixture obtained in step b) and adjusting the pH within the range of 6 to 8 by addition of organic acid, preferably citric acid, citrate or mixtures thereof, in an amount of at least 2.5 wt % based on dry weight of the composition, and
d)-sterilising the blend.

Addition of minerals and vitamins can be made either by sterilising the minerals and vitamins ingredients separately from the other components part of the composition and incorporating the sterilised minerals and vitamins to the sterilised composition or adding the minerals and vitamins with the other components according to the process above mentioned, the sterilisation of the minerals and vitamins being made when the composition is sterilised.

Examples of enteral feedings are feedings that can be administered via a tube to a patient, for instance undergoing cardiosurgery, containing 0.5 % to 7 %(w/v), preferably 1 to 5 %(w/v), more preferably 1 to 4 %(w/v) phospholipids in the presence of proteins, oils, sugars, fibrous and other components which are normally present in a complete enteral feeding. The feeding is started 24 hours before the surgery, which depending on the condition of the patient can be either by sip-feeding or tube-feeding, and is continued during 24 to 72 hours after the surgery via tube. Tube-feeding can also be carried out directly into the duodenum or jejunum, thereby advantageously maintaining an empty stomach and preventing aspiration during surgery. Continuous treatment with the invention composition is an important and preferred aspect of the present invention. Indeed, continuous treatment, i.e. starting before operation, if any, and continuing for 4 to 5 days, provides a sustained production of chylomicrons, thereby providing a better and long lasting resistance against microbial infections, in particular those giving rise to sepsis.

A capsule or powdered food supplement containing the lipid mixture according to the invention is given to people with inflammatory bowel disease. The supplement may further contain fibers, oligosaccharides, vitamins, in particular fat soluble vitamins, as indicated above, probiotics, anti-oxidants, herbal or plant extracts, proteins or peptides, etc. The supplement is given to patients in remission in order to prevent recurrence of inflammation or to alleviate the inflammation once it re-occurs.

A liquid sip feeding with a phospholipid concentration of 1-7 % (w/v), preferably 1-6 % (w/v), even more preferably 1-5 % (w/v), can for instance be administered to a patient with obstructive jaundice, who will undergo surgery. Proteins and fats are present but polysaccharides and micronutrients may also be present. The feeding is administered 24-12 hours before surgery and continues as soon as possible after surgery for 24 to 72 hours.

The composition of the invention is also suitable for newborn and premature children as well as animals.

The following are examples illustrating the present invention:

**In the examples, the following abbreviations have been used**

| | |
|---|---|
| Protein 1 | mixture of casein derived form cow's milk and wheat protein hydrolysate in weight ratio of 0.75:1 |
| Protein 2 | Casein derived from cow's milk |
| Protein 3 | Cow's milk protein |
| Carbohydrates 1 | mixture of glucose, maltose and maltodextrin in weight ratio of 1:3.5:55.5 |
| Carbohydrates 2 | mixture of glucose, maltose and maltodextrin in weight ratio of 1:3.5:5.5 |
| Carbohydrates 3 | mixture of glucose, maltose, saccharose and maltodextrin in weight ratio of 1:2.7:12:42.7 |
| Carbohydrates 4 | mixture of glucose, maltose and maltodextrin in weight ratio of 1:9.3:28 |
| Phospholipids | Derived from Soy lecithin |
| Triglycerides 1 | Vegetable oil consisting mainly of Canola oil |
| Triglycerides 2 | Derived from soy |

The composition of the fatty acid in the triglycerides 1 present in the examples below is of 7-10% saturated fatty acid, 60-65% monounsaturated and 28-31% of polyunsaturated fatty acids. The weight ratio of n3:n6 fatty acid in triglyceride 1 is of 5:1. In triglyceride 2 the amount of saturated, monounsaturated and polyunsaturated fatty acids is about 71,18 and 61, respectively, with a n3:n6 ratio of 7.7. Triglycerides 1-2 are long chain fatty acid triglycerides.

### Example 1

Tube feeding containing per 100 ml

| | |
|---|---|
| Energy | 125 kcal |
| Protein 1 | 7.5 g |
| Carbohydrates 1 | 14.5 g |
| Lipids | 4.2 g including: |
| Phospholipids | 2.1 g |
| Triglycerides 1 | 0.66 g |
| Triglycerides 2 | 1.44 g |

Vitamins, minerals and trace elements and other components as known in the art of tube feeding, for instance Na, K, Cl, Ca, Mg, Fe, Se, Cu, P, Zn, I, Vit A, D, C, E, , B6, B12, K, folic acid, pantothenic acid, niacin, biotin, riboflavin, folic acid, choline, myoinositol, L-carnithine in amounts known in the art of tube feeding can also be included in the above mentioned composition and in those of examples 2 - 6.

### Example 2

Tube feeding containing per 100 ml

| | |
|---|---|
| Energy | 100 kcal |
| Protein 2 | 4.0 g |
| Carbohydrates 2 | 12.3 g |
| Lipids | 3.9 g. including: |
| Phospholipids | 2.0 g |
| Triglycerides 1 | 0.63 g |
| Triglycerides 2 | 1.27 g |
| Fibre | 1 g |

### Example 3

Liquid feeding (sip feeding) to be used as a supplement containing per 100 ml

| | | |
|---|---|---|
| Energy | 100 kcal | |
| Protein 2 | 4 g | |
| Carbohydrates 3 | 8.8 g | |
| Lipids | 5.4 g including: | |
| Phospholipids | 3.6 g Triglycerides 1 | 1.19 g |
| Triglycerides 2 | 0.61 g | |

### Example 4

Liquid feeding (sip feeding) to be used as a supplement containing per 100 ml

| | |
|---|---|
| Energy | 130 kcal |
| Protein 2 | 2.8 g |
| Carbohydrates 3 | 13.3 g |
| Lipids | 7.3 g including: |
| Phospholipids | 5.0 g |
| Triglycerides 2 | 2.3 g |

### Example 5

Nutritional oral supplement in powder form. To be diluted according individual energy/fluid needs. Composition per 100 g powder

| | |
|---|---|
| Energy | 460 kcal |
| Protein 2 | 18.6 g |
| Carbohydrates 4 | 52.3 g |
| Lipids | 18.1 g including: |
| Phospholipids | 10.8 g ratio |
| Triglycerides 1 | 3.38 g g |
| Triglycerides 2 | 3.92 g |

### Example 6:

A nutrition intended for tube feeding containing per 100 ml:

| | |
|---|---|
| Energy | 125 kcal |
| Protein 1 | 7.5 g |
| Carbohydrates 2 | 12.3 g |
| Lipids | 6.67 g. including: |
| Phospholipids | 4.58 g |
| Triglycerides 2 | 2.09 g |
| Fibre (optionally) | 1.5 g |

### Example 7:

### Animals

The protocol of this study was approved by the Animal Care Committee of the University of Maastricht, the Netherlands. Healthy male Sprague-Dawley rats, weighing 301 - 410 grams (average, 342 grams) purchased from Charles River (Maastricht, the Netherlands) were housed under controlled conditions of temperature (20-22°C) and humidity (50%). Before the beginning of the experiments, rats were fed water and chow ad libitum.

### Experimental Design

Animals were divided into six groups (n=8 per group). Control rats (C) were starved for 18 hours and sacrificed at t=24 h to assess the effect of fasting alone. Sham shock (SS) rats were starved for 18 h and at t=0 the femoral artery was cannulated, but no shock was induced. The haemorrhagic shock groups were either starved 18 hours before the procedure (HS-S), or enterally fed with a low-fat liquid enteral diet (HS-LF), a high-fat high triglyceride liquid enteral diet (HS-HT) or a high-fat high phospholipid liquid enteral diet (HS-HP) via oral gavage at 18 h (3 ml), 2 h (0.75 ml) and 45 min (0.75 ml) before the haemorrhagic shock was induced, and at 3 h (0.75 ml) and 6 h (0.75 ml) after the shock. Blood and tissue samples were taken at 24 hours after onset of shock. The amount of fat in the low-fat diet was isocalorical to that present in standard rodent chow (16.0 energy %). The high-fat liquid enteral diets were isocaloric and isonitrogenous to the low-fat diet, but contained 52.2 energy % fat. The composition of the diets is given in table 2.

In a second experiment the high phospholipid and high triglyceride diets were tested in rats receiving a haemorrhagic shock. The two experiments were combined, so that the HS-TG and HS-PL group now consisted of 16 animals.

**Table 2: Composition of the food fed to the rats (per 100 ml)**

| Diet: | Low Fat (HS-LF) | High triglyceride (HS-HT) | High Phospholipid (HS-HP) |
|---|---|---|---|
| Energy kcal | 130 | 130 | 130 |
| Carbohydrates 2 | 24.47 g (75.3%) | 13.30 g (40.9%) | 13.30 g |
| Proteins 3 | 2.81 g (8.6%) | 2.81 g (8.6%) | 2.81 g |
| Fat g | 2.31 g (16.0%) | 7.28 g (50.4%) | 7.28 g |
| Triglycerides 1 | 2.31 g | 7.28 g | |
| Triglycerides 2 | | | 2.28 g |
| Phospholipids | | | 5.00 g |

### Haemorrhagic shock procedure

Rats were anesthetised with intraperitoneally injected sodium pentobarbital (40 mg/kg). The skin over the left femoral area was shaved and desinfected with povidone iodine solution. The animals were placed in the supine position and allowed to breathe spontaneously. During surgery and throughout the experiment, body temperature was maintained at 37°C with an infrared heating lamp controlled by a thermo analyser system (Hugo Sachs Elektronik, March-Hugstetten, Germany) connected to a rectal probe. The femoral artery was dissected using aseptic technique and cannulated with polyethylene tubing (PE-10) containing heparinised saline (10 IU/ml). Arterial blood pressure was continuously measured (Uniflow^{tm} external pressure transducer; Baxter^{™}, Utrecht, the Netherlands) and recorded as Mean Arterial Pressure (MAP). Heart rate (HR) was continuously assessed from the instantaneous pressure signal. To keep the arterial catheter patent, it was constantly perfused with physiological saline (3ml/h) via the Uniflow^{tm} system; no heparin was used.

After an acclimatisation period of 30 minutes, rats were subjected to haemorrhage by withdrawing blood in quantities of 2.1 ml/100 gram of body weight (representing approximately 30-40% of the circulating volume) at a rate of 1 ml/minute. At 50 minutes after the induction of shock, the catheter was removed and the femoral artery ligated.

Six hours after haemorrhage, they were allowed access to standard chow ad libitum. Rats in the sham-shock group were anesthetised and the left femoral artery was cannulated. Sham shock rats were monitored similar as the haemorrhagic shock group, however no blood was withdrawn.

Twenty-four hours after induction of shock, the rats were anesthetised with sodium pentobarbital (60 mg/kg). The skin over de abdomen was shaved and desinfected with povidone iodine. The abdomen was opened via a midline incision, blood samples were taken and mesenteric lymph nodes, the midsection of the spleen and segment IV of the liver were aseptically removed for bacteriological examination.

### Bacterial Translocation

Mesenteric lymph nodes (MLN), the mid-section of the spleen and a segment of the liver were collected aseptically in 2 ml pre-weighed thioglycolate broth tubes (Becton Dickinson (BBL) Microbiology Europe, Maylan, France). After weighing, the tissue specimens were homogenised with sterile grinding rods (Potter S, B.Braun Melsungen, Melsungen, Germany). Subsequently, 500 µl-volumes were transferred onto the following agar plates: Columbia III blood agar base supplemented with 5% vol/vol sheep blood (BBL) (duplicate plates), Chocolate PolyviteX agar (BioMérieux, Marcy L'Etoile, France), and Schaedler Kanamycin-Vancomycin agar supplemented with 5% sheep blood (BBL). Aliquots were spread over the entire surface of the agar. All agar plates were incubated for 48h, in a 5% CO₂-enriched atmosphere or under anaerobic conditions (Shaedler agar plates). After incubation, the colonies were counted on the non-selective Columbia sheep blood agar plates. For determination of the number of colony forming units (CFU) per gram tissue, the number of colonies was counted on all aerobic plates and next adjusted to the weight of the grounded tissue.

### Statistical analysis

The 1-sided Mann-Whitney U test was used for between-group comparisons, since the hypothesis was that phospholipids were better than triglycerides. For translocation incidence a 1-sided Fisher's Exact test was used, using a cut off of 50 cfu/gram tissue. A value of 50 or lower is regarded as no translocation having occurred, a value higher than 50 was regarded as bacterial translocation having occurred. P ≤ 0.05 was considered statistically significant.

### Results

Table 3 shows the results regarding the bacterial numbers translocated, wherein the mean value and range (between brackets) are presented.

**Table 3: Bacterial numbers translocated as mean value (range) obtained from 1-sided Mann-Whitney U test**

| | **MLN** | **Spleen** | **Liver** | **Total** |
|---|---|---|---|---|
| **First experiment** | | | | |
| C (n=8) | 0 | 0 | 0 | o^{**/##} |
| SS (n=8) | 8 (0-33) | 2(0-17) | 12(0-95) | 22^{**/##} |
| HS-S (n=8) | 212(60-483) | 12 (0-998) | 44 (0-249) | 327 |
| HS-LF (n=8) | 86 (30-209) | 66 (7-2543) | 28 (0-1416) | 261 1 |
| HS-HT (n=8) | 3 (0-144) | 0 (0-91) | 0 (0-115) | 25^{**/##} |
| HS-HP (n=8) | 0 (0-13) | 0 (0-6) | 0 | 0^{****/*##*/*§*} |

| **Combined experiment** | | | | |
|---|---|---|---|---|
| HS-HT (n=16) | 10 (0-550) | 3(0-308) | 0 (0-115) | 83^{*/#} |
| HS-HP (n=16) | 0 (0-77) | 0 (0-73) | 0 (0-124) | 0^{**/##/¶} |

| | | | | |
|---|---|---|---|---|
| * p < 0.05 and ** p < 0.01 compared toHS-LF ^{#} p < 0.05 and^{##} p < 0.01 compared to HS-S § p = 0.07 compared to HS-TG ¶p = 0.05 compared to HS-TG | | | | |

In the first experiment, starved rats receiving a haemorrhagic shock (HS-S) and rats fed an isocaloric low fat diet receiving a haemorrhagic shock (HS-LF) showed significantly higher bacterial translocation numbers compared to control starved rats (C), or starved rats receiving a sham shock (SS). Rats receiving a high fat diet showed significant less bacterial translocation compared to the other groups receiving a haemorrhagic shock. In the first experiment the group fed high concentration of phospholipids (HS-HP) showed a strong tendency for lower bacterial numbers translocated compared to the group fed high concentration of triglycerides (HS-HT) (p=0.07). Upon extending these groups to n=16 after performing a second experiment, the differences obtained between the HS-HT and HS-HP group were significant (P=0.05) with a lower translocation in the HS-HP group.

### Translocation incidence

Table 4 shows the results for incidence of bacterial translocation. Starved control rats (C) and sham shocked rats (SS) showed less incidence of translocation compared to the starved or low fat fed rats undergoing a haemorrhagic shock. A diet with high fat had a positive effect on the incidence of bacterial translocation. Of these high fat diets a significant effect of a diet with a high concentration of phospholipids (HS-HP) compared to a diet with a high concentration of triglycerides (HS-HT) on incidence of bacterial translocation was observed.

The results obtained in these experiments demonstrate that a high fat diet protects against bacterial translocation occurring as a result from surgery and haemorrhage both on the level of incidence as on the level of bacterial numbers translocated. From the two high fat diets tested, the diet with an high ratio phospholipids to triglycerides showed a significant better protective effect than a high fat diet comprising triglycerides alone. Reduction of incidence of bacterial translocation and reduction in the number of bacteria translocated reduces the change on bacteraemia, and reduces the influx of LPS and LTA and thereby thus the chance for development of endotoxaemia and sepsis.

**Table 4 : Translocation incidence obtained from 1-sided Fisher's Exact test**

| | **First Experiment** | **Second Experiment** | **Combined (n=16)** |
|---|---|---|---|
| C | 0/8 | | |
| SS | 1/8 | | |
| HS-S | 8/8 | | |
| HS-LF | 8/8 | | |
| HS-HT | 4/8 (50%) | 6/8 (75%) | 10/16 (62.5%) |
| HS-HP | 0/8 (0%) | 4/8 (50%) | 4/16 (25%) |
| 1-sided. | p = 0.04 | P = 0.15 | p = 0.04 |

## Claims

1. Use of a composition comprising proteins and lipids, the lipids comprising phospholipids and triglycerides in a weight ratio of phospholipids to triglycerides greater than 1, and wherein the composition comprises less than 0.5 % by weight of cholesterol or precursors thereof, for the manufacture of a nutritional composition for the treatment and/or prevention of endotoxaemia, sepsis, or bacteraemia by enteral administration.

2. Use according to claim 1, wherein the phospholipids are administered in a dose of 0.008 to 2.0 gram per kg body weight per day.

3. Use according to claim 1 or 2, wherein the triglycerides are administered in a dose of 0.01 to 1.5 g per kg body weight per day.

4. Use according to any one of the preceding claims, wherein the composition further contains one or more fat soluble substances preferably selected from vitamin K (menaquinones), ubiquinones, carotenoids such as vitamin A, conjugated linoleic acid, lipoic acid, vitamin D and mixtures thereof.

5. Use according to any one of the preceding claims, where sepsis, endotoxaemia and/or bacteraemia is associated with major surgery, critical illness, Inflammatory Bowel disease (IBD), HELLP syndrome, an enhanced risk for bacterial translocation and sepsis in general, in particular major trauma, burns, pneumonia, especially caused or complicated by bacteria, decubitus, during radio/chemo therapy, with a compromised immune system or with an obstructed bile duct.

6. Use according to any one of the preceding claims for the treatment of persons that do not allow intake of a large volume, in particular patients suffering from anorexia nervosa, cancer or AIDS patients and elderly.

7. Use according to any one of the preceding claims for the treatment of neonates.

8. Use according to any one of claims 1 - 4 for the manufacture of a nutritional composition for the treatment of farm animals before they are slaughtered or weaning pigs.

9. An enteral composition comprising:
a)- at least 1 % by weight of proteins and/or peptides, and
b)- at least 3.5 % by weight of lipids, the lipid fraction comprising phospholipids and triglycerides in a weight ratio of phospholipids to triglycerides greater than 1, wherein the composition comprises less than 0.5 % by weight of cholesterol or precursors thereof, wherein said phospholipids are present in an amount of at most 78 wt% of the lipid fraction, and wherein the triglycerides contain at least 50 wt% long chain fatty acids.

10. An enteral composition according to claim 9, wherein the phospholipids comprise phosphatidylcholine and one or more of phosphatidylethanolamine, phosphatidylinositol phosphatidyl serine, phosphatidyl glycerol and phosphatidic acid.

11. An enteral composition according to claim 9 or 10, wherein the phospholipid fraction comprises at most 72 % of phosphatidyl choline, less than 70 % phosphatidyl ethanolamine, and more than 5 % of negatively charged phospholipids, based on the weight of the total amount of phospholipids present in the composition.

12. An enteral composition according to any one of claims 9-11, wherein the triglycerides contain more than 60 % and most preferably more than 75 % long chain fatty acids, in particular long chain polyunsaturated fatty acids.

13. An enteral composition according to any one of claims 9-12, wherein the weight ratio of phospholipids to triglycerides is greater than 1.5, preferably greater than 2.

14. An enteral composition according to any one of claims 9-13, wherein the composition comprises less than 0.2 % by weight of cholesterol or precursors thereof.

15. An enteral composition according to any one of claims 9 - 14, wherein the proteinaceous material comprises at least 30 % by weight of the composition of proteins and/or peptides having a molecular weight of at least 0.3k Daltons.

16. An enteral composition according to any one of claims 9 - 15, wherein the composition contains egg or an egg fraction.

17. An enteral composition according to any one of claims 9 - 16, wherein the composition further contains immunoglobulins, preferably IgY.

18. An enteral composition according to any one of claims 9-17, wherein the composition contains soy lecithin.

19. An enteral composition according to any one of claims 9 - 18, wherein the composition is a tube feeding.

20. An enteral composition according to any one of claims 9 - 19, wherein the composition is substantially free of cholesterol or precursors thereof.

21. An enteral composition according to any one of claims 9 - 20, wherein the composition further contains one or more fat soluble substances, preferably selected from vitamin K (menaquinones), ubiquinones, carotenoids such as vitamin A, conjugated linoleic acid, lipoic acid, vitamin D and mixtures thereof.

22. An enteral composition according to any one of claims 9-21, wherein the composition further contains one or more organic acids, preferably selected from citric acid, malic acid, lactic acid, salts thereof, and mixtures thereof.

23. A process for producing a composition as defined in any one of claims 9 - 22, wherein the process comprises the steps of:
a)-mixing the non-lipid ingredients together and adjusting the pH within the range of 6 to 8;
b)-incorporating the lipid ingredient in the mixture obtained in step a) by mixing;
c)-pasteurising the mixture obtained in step b) and adjusting the pH within the range of 6 to 8 by addition of organic acid, preferably citric acid, citrate or mixtures thereof, in an amount of at least 2.5 wt.% based on dry weight of the composition, and
d)-sterilising the mixture.

## Patentansprüche

1. Verwendung einer Zusammensetzung, umfassend Proteine und Lipide, wobei die Lipide Phospholipide und Triglyceride in einem Gewichtsverhältnis von Phospholipiden zu Triglyceriden größer als 1 enthalten, und wobei die Zusammensetzung weniger als 0,5 Gew.-% Cholesterin oder Präkursoren (Vorstufen) hiervon enthält, zur Herstellung einer Nährstoffzusammensetzung zur Behandlung von und/oder zur Vorbeugung gegen Endotoxämie, Sepsis oder Bakteriämie durch enterale Verabreichung.

2. Verwendung nach Anspruch 1, wobei die Phospholipide in einer Dosis von 0,008 bis 2,0 Gramm pro kg Körpergewicht je Tag verabreicht werden.

3. Verwendung nach Anspruch 1 oder 2, wobei die Triglyceride in einer Dosis von 0,01 bis 1,5 g pro kg Körpergewicht je Tag verabreicht werden.

4. Verwendung nach einem der vorhergehenden Ansprüche, wobei die Zusammensetzung des Weiteren ein oder mehr fettlösliche Substanzen enthält, bevorzugt ausgewählt aus Vitamin K (Menachinone), Ubichinone, Carotenoide wie Vitamin A, konjugierter Linolsäure, Liponsäure, Vitamin D und Mischungen hieraus.

5. Verwendung nach einem der vorhergehenden Ansprüche, wobei Sepsis, Endotoxämie und/oder Bakteriämie in Zusammenhang stehen mit großen chirurgischen Eingriffen, kritischer Erkrankung, entzündlicher Darmerkrankung, HELLP-Syndrom, einem erhöhten Risiko bakterieller Translokation und Sepsis im Allgemeinen, insbesondere großem Trauma, Verbrennungen, Pneumonie, hauptsächlich verursacht oder verschlimmert durch Bakterien, Dekubitus, während einer Strahlen-/Chemotherapie, bei einem geschwächten Immunsystem oder einem versperrten Gallengang.

6. Verwendung nach einem der vorhergehenden Ansprüche zur Behandlung von Personen, die die Aufnahme eines großen Volumens nicht zulassen, insbesondere Patienten, die an Magersucht leiden, Krebs- oder Aids-Patienten und ältere Menschen.

7. Verwendung nach einem der vorhergehenden Ansprüche zur Behandlung von Neugeborenen.

8. Verwendung nach einem der Ansprüche 1 - 4 zur Herstellung einer Nährstoffzusammensetzung zur Behandlung von landwirtschaftlichen Nutztieren, bevor sie geschlachtet werden, oder für abgesäugte Schweine.

9. Enteralzusammensetzung umfassend:
a) - mindestens 1 Gew.-% Proteine und/oder Peptide, und
b) - mindestens 3,5 Gew.-% Lipide, wobei der Lipidanteil Phospholipide und Triglyceride in einem Gewichtsverhältnis von Phospholipiden zu Triglyceriden größer als 1 enthält, wobei die Zusammensetzung weniger als 0,5 Gew.-% Cholesterin oder Vorstufen hiervon enthält, wobei die Phospholipide in einer Menge von höchstens 78 Gew.-% des Lipidanteils vorhanden sind, und wobei die Triglyceride wenigstens 50 Gew.-% langkettige Fettsäuren enthalten.

10. Enteralzusammensetzung nach Anspruch 9, wobei die Phospholipide Phosphatidylcholin und ein oder mehr Phosphatidyl- ethanolamin, Phosphatidylinositol-Phosphatidylserin, Phosphatidylglycerol und Phosphatidsäure enthalten.

11. Enteralzusammensetzung nach Anspruch 9 oder 10, wobei der Phospholipidanteil höchstens 72% an Phosphatidylcholin, weniger als 70% Phosphatidylethanolamin und mehr als 5% negativ geladener Phospholipide enthält, basierend auf dem Gewicht der Gesamtmenge an Phospholipiden, die in der Zusammensetzung vorhanden sind.

12. Enteralzusammensetzung nach einem der Ansprüche 9 - 11, wobei die Triglyceride mehr als 60% und am bevorzugten mehr als 75% langkettige Fettsäuren enthalten, insbesondere langkettige mehrfach ungesättigte Fettsäuren.

13. Enteralzusammensetzung nach einem der Ansprüche 9 - 12, wobei das Gewichtsverhältnis von Phospholipiden zu Triglyceriden größer als 1,5, bevorzugt größer als 2 ist.

14. Enteralzusammensetzung nach einem der Ansprüche 9 - 13, wobei die Zusammensetzung weniger als 0,2 Gew.-% Cholesterin oder Vorstufen hiervon enthält.

15. Enteralzusammensetzung nach einem der Ansprüche 9 - 14, wobei das proteinöse Material mindestens 30 Gew.-% der Zusammensetzung aus Proteinen und/oder Peptiden mit einem Molekulargewicht von wenigstens 0,3k Dalton enthält.

16. Enteralzusammensetzung nach einem der Ansprüche 9 - 15, wobei die Zusammensetzung Ei oder einen Eianteil enthält.

17. Enteralzusammensetzung nach einem der Ansprüche 9 - 16, wobei die Zusammensetzung des Weiteren Immunglobuline enthält, vorzugsweise IgY.

18. Enteralzusammensetzung nach einem der Ansprüche 9 - 17, wobei die Zusammensetzung Sojalecithin enthält.

19. Enteralzusammensetzung nach einem der Ansprüche 9 - 18, wobei die Zusammensetzung mittels Sonde verabreicht wird.

20. Enteralzusammensetzung nach einem der Ansprüche 9 - 19, wobei die Zusammensetzung frei von Cholesterin oder Vorstufen hiervon ist.

21. Enteralzusammensetzung nach einem der Ansprüche 9 - 20, wobei die Zusammensetzung des Weiteren eine oder mehr fettlösliche Substanzen enthält, bevorzugt ausgewählt aus Vitamin K (Menachinone), Ubichinone, Carotenoide wie Vitamin A, konjugierter Linolsäure, Liponsäure, Vitamin D und Mischungen hieraus.

22. Enteralzusammensetzung nach einem der Ansprüche 9 - 21, wobei die Zusammensetzung des Weiteren ein oder mehr organische Säuren enthält, bevorzugt ausgewählt aus Zitronensäure, Apfelsäure, Milchsäure, Salze hieraus und Mischungen hieraus.

23. Verfahren zur Herstellung einer Zusammensetzung wie in einem der Ansprüche 9 - 22 definiert, wobei das Verfahren die Schritte umfasst:
a)- Miteinandervermischen der nicht lipiden Bestandteile und Einstellen des pH-Werts im Bereich von 6 bis 8;
b)- Einarbeiten des Lipidbestandteils in das in Schritt a) gewonnene Gemisch durch Vermischen;
c)- Pasteurisieren der in Schritt b) erzielten Mischung und Einstellen des pH-Werts im Bereich von 6 bis 8 durch Zugeben von organischer Säure, bevorzugt Zitronensäure, Citrat oder Mischungen hieraus, in einer Menge von wenigstens 2,5 Gew.-% basierend auf dem Trockengewicht der Zusammensetzung, und
d)- Sterilisieren der Mischung.

## Revendications

1. Utilisation d'une composition comprenant des protéines et des lipides, les lipides comprenant des phospholipides et des triglycérides dans un rapport pondéral de phospholipides par rapport aux triglycérides supérieur à 1, et dans laquelle la composition comprend moins de 0, 5% en poids de cholestérol ou de précurseurs de celui-ci, pour la préparation d'une composition nutritionnelle pour le traitement et/ou la prévention d' endotoxaemia, de sepsis, ou de bactéraemia par administration entérale.

2. Utilisation selon la revendication 1, dans laquelle les phospholipides sont administrés dans une dose de 0,008 à 2,0 g/kg de poids corporel par jour.

3. Utilisation selon la revendication 1 ou 2, dans laquelle les triglycérides sont administrés dans une dose de 0,01 à 1,5g/kg de poids corporel par jour.

4. Utilisation selon l'une quelconque des revendications précédentes, dans laquelle la composition comprend en outre une ou plusieurs substances grasses solubles de préférence choisies parmi la vitamine K (ménaquinones), ubiquinone, caroténoides, telles que vitamine A, acide linoléique conjugué, acide lipoïque, vitamine D et des mélanges de ceux-ci.

5. Utilisation selon l'une quelconque des revendications précédentes dans laquelle le sepsis, l'endotoxaemia et/ou bactéraemia est associé avec une chirurgie majeure, une maladie critique, la maladie inflammatoire de l'intestin (IBD), le syndrome de HELLP, un risque augmenté de translocation bactérienne et la septicémie en général, en particulier les traumatismes majeurs, les brûlures, la pneumonie en particulier causée ou compliquée par des bactéries, le décubitus, la radio ou chimiothérapie, avec un système immuno compromis ou avec un canal biliaire obstrué.

6. Utilisation selon l'une quelconque des revendications précédentes pour le traitement de personnes qui sont interdites d'absorber un grand volume, en particulier des patients souffrant d'anorexie nerveuse, de cancer ou de SIDA ou des patients âgés.

7. Utilisation selon l'une quelconque des revendications précédentes pour le traitement des néonés.

8. Utilisation selon l'une quelconque des revendications 1 à 4, pour la préparation d'une composition nutritionnelle pour le traitement des animaux de fermes avant leur abattage ou des porcs sevrés.

9. Composition entérale comprenant :
a)- au moins 1% en poids de protéines et/ou de peptides, et
b)- au moins 3,5% en poids de lipides, la fraction de lipides comprenant des phospholipides et des triglycérides dans un rapport pondéral de phospholipides par rapport aux triglycérides supérieur à 1, cette composition comprenant moins de 0,5% en poids de cholestérol ou de précurseurs de celui-ci, lesdites phospholipides étant présentes dans une quantité au plus égale à 78% en poids de la fraction de lipides et les triglycérides comprenant au moins 50% en poids d'acide gras à longue chaîne.

10. Composition entérale selon la revendication 9, dans laquelle les phospholipides comprennent du phosphatidylcholine, et une ou plusieurs phosphatidyléthanolamine, phosphatidylinositol, phosphatidylsérine, phosphatidylglycérol et acide phosphatidique.

11. Composition entérale selon la revendication 9 ou 10 dans laquelle la fraction phospholipidique comprend au plus 72% de phosphatidylcholine, moins de 70% de phosphatidyléthanolamine et plus de 5% de phospholipide chargé négativement, sur la base du poids de la quantité de poids total de phospholipides présents dans la composition.

12. Composition entérale selon l'une quelconque des revendications 9 à 11, dans laquelle les triglycérides contiennent plus de 60% et de préférence plus de 75% d'acide gras à longue chaîne en particulier des acides gras polyinsaturés à longue chaîne.

13. Composition entérale selon l'une quelconque des revendications 9 à 12, dans laquelle le rapport pondéral des phospholipides sur les triglycérides est supérieur à 1,5, de préférence supérieur à 2.

14. Composition entérale selon l'une quelconque des revendications 9 à 13, dans laquelle la composition comprend moins de 0,2% en poids de cholestérol ou de précurseurs de celui-ci.

15. Composition entérale selon l'une quelconque des revendications 9 à 14, dans laquelle la matière protéineuse comprend au moins 30% en poids de la composition, de protéines et/ou de peptides ayant un poids moléculaire d'au moins 0,3k Daltons.

16. Composition entérale selon l'une quelconque des revendications 9 à 15, dans laquelle la composition contient de l'oeuf ou une fraction d'oeuf.

17. Composition entérale selon l'une quelconque des revendications 9 à 16, dans laquelle la composition contient en outre des immunoglobulines, de préférence des IgY.

18. Composition entérale selon l'une quelconque des revendications 9 à 17, dans laquelle la composition contient de la lécithine de soja.

19. Composition entérale selon l'une quelconque des revendications 9 à 18, dans laquelle la composition est une alimentation par tube.

20. Composition entérale selon l'une quelconque des revendications 9 à 19, dans laquelle la composition est sensiblement dépourvue de cholestérol ou de précurseurs de celui-ci.

21. Composition entérale selon l'une quelconque des revendications 9 à 20, dans laquelle la composition comprend en outre une ou plusieurs substances grasses solubles, de préférence choisies parmi la vitamine K (ménaquinones), ubiquinones, caroténoides telle que la vitamine A, l'acide linoléique conjugué, l'acide lipoique, la vitamine D et des mélanges de ceux-ci.

22. Composition entérale selon l'une quelconque des revendications 9 à 21, dans laquelle la composition contient en outre un ou plusieurs acides organiques, de préférence choisi parmi l'acide citrique, l'acide malique, l'acide lactique, des sels de ceux-ci et des mélanges de ses derniers.

23. Procédé pour préparer une composition telle que définie dans l'une quelconque des revendications 9 à 22, ce procédé comprenant les étapes suivantes :
a)- mélanger ensemble les ingrédients non-lipidiques et ajuster le pH dans l'intervalle de 6 à 8 ;
b) incorporer l'ingrédient lipidique dans le mélange obtenu dans l'étape a) sous agitation ;
c)- pasteuriser le mélange obtenu dans l'étape b) et ajuster le pH dans l'intervalle de 6 à 8 par addition d'acide organique de préférence d'acide citrique, de citrate ou de mélange de ceux-ci, dans une quantité d'au moins 2,5% en poids basé sur le poids sec de la composition, et
d)- stériliser le mélange.
